# EUROPEAN PATENT APPLICATION

(11) **EP 4 327 856 A1**
(43) Date of publication of application: **28.02.2024**
(21) Application number: 22191706.5
(22) Date of filing: 23.08.2022
(51) Int. Cl.: A61M 39/28, A61M 39/08

(54) **CRIMPING SLEEVE**

(71) Applicant: Single Use Support GmbH, 6330 Kufstein (AT)
(72) Inventor: HÖRFARTER, Christoph, 6341 Ebbs (AT)
(74) Representative: Torggler & Hofmann Patentanwälte - Innsbruck

(57) **Abstract**

Crimping sleeve for crimping, in particular sealing, a tube (2), in particular for crimping a tube (2) for conveying a biopharmaceutical fluid, wherein the crimping sleeve (1) comprises a first component part (3,13) and a separate second component part (4,14)and the first component part (3,13) and the second component part (4,14) can be engaged with the tube (2) radially from different sides.

## Description

The present invention concerns a crimping sleeve for crimping, in particular sealing, a tube, in particular for crimping a tube for conveying a biopharmaceutical fluid, and a method for crimping, in particular sealing, a tube, in particular for crimping a tube for conveying a biopharmaceutical fluid.

During the biopharmaceutical production process, it is common to create biopharmaceutical fluids and to store them in bulk storage containers. For transportation, distribution, or storing the biopharmaceuticals it is also known to fill the biopharmaceutical into smaller containers which can be handled individually.

It is to be avoided to contaminate the biopharmaceuticals in any way. Therefore, the containers have to be kept sterile, and their sterile integrity has to be maintained. The same is true for tubes which are used to transfer the biopharmaceutical from the bulk source to the container.

In order to be able to separate the container from the tube it is therefore known to crimp the tube near the container. The container and a part of the tube can be sealed in a sterile way. The tube can subsequently be severed on the other side of the crimp without disturbing the sterile integrity of the container and said part of the tube.

As is evident, just this filling process of the containers comprises many steps which have to be followed meticulously. Otherwise, the sterile integrity of the biopharmaceutical may be in jeopardy. Considering the extreme value biopharmaceutical fluids can attain because of the complex, time consuming, and elaborate production process, it is evident that a simplified procedure for filling the containers would be highly desirable.

It is the object of the invention to provide an apparatus and a method, which allow for a simplified filling of containers with a biopharmaceutical fluid.

Regarding the apparatus this object is achieved with the features of claim 1, providing a crimping sleeve which comprises a first component part and a separate second component part, wherein the first component part and the second component part can be engaged with the tube radially from different sides.

Regarding the method the object is achieved with the features of claim 14, wherein at least the following steps are carried out:
- radially engaging a first component part of a crimping sleeve with the tube,
- radially engaging a second component part of the crimping sleeve with the tube from a different side than the first component part, and
- exerting a crimping action onto the crimp sleeve, thereby crimping - and preferably sealing - the tube.

In a basic aspect of the invention a first component part and a separate second component part, which can be engaged with the tube radially from different sides, allow for engaging the crimping sleeve with the tube after the filling process.

Crimping sleeves according to the prior art have to be threaded onto the tube before the tube is connected to the container or a bulk source, i.e., many steps of the filling procedure earlier than the actual crimping action.

Since the tube must be sterile the crimping sleeve either has to be sterilised together with the tube or the crimping sleeve has to be threaded onto the tube after the sterilising process, e.g. when the tube is taken out of its sterile packaging. According to the prior art it was believed that these two are the only alternatives, both of which introduce their own sorts of complications.

Either the sterilisation process of the tube is made more difficult because of the presence of the metal sleeve, or the crimping sleeve has to be threaded onto the tube interfering with the connectors which also have to be kept sterile. In practice, there was therefore no alternative to sterilising the crimping sleeve at some point.

Using a crimping sleeve and/or a method according to the invention all of these complications can be avoided in a manner which is elegant from a technical point of view.

In preferred embodiments the fluid is a liquid, in particular a biopharmaceutical liquid.

Biopharmaceutical fluids and/or liquids can be understood as fluids or liquids which occur in the biopharmaceutical production process. They can either be end products (i.e., the actual medicine) or intermediary products of the production process.

Biopharmaceutical fluid or liquids can for example be protein solutions, end products from a purification procedure, antibody solutions and other high-value intermediate products in the pharmaceutical production cycle, and naturally the pharmaceuticals themselves.

The crimping sleeve according to the invention can be used with an inner crimping sleeve according to the invention or not according to the invention. The crimping sleeve then takes the role of an intermediary crimping sleeve. In this case the first component part and the second component part can be called first intermediary component and second intermediary component.

The crimping sleeve and/or the first component part and/or the second component part and/or the first intermediary part and/or the second intermediary part can directly or indirectly engage with the tube. In what follows it will be referred to engagement with the tube. However indirectly engaging with the tube via an inner crimping sleeve is in each case also meant as an option.

The crimping sleeve according to the invention can preferably be configured to undergo plastic deformation under crimping action, such as a crimping force acting on it.

The plastic deformation preferably seals the tube.

If the crimping sleeve is an intermediary sleeve, it can be configured to not undergo plastic deformation, but to exert the crimping action onto the inner crimping sleeve according to the invention or not according to the invention.

The tube can for example be a flexible tube and/or can be made from silicone.

In preferred embodiment the container can be a Single Use Bag. Single Use Bags are bags made of two or more layers of plastic which are laminated, welded, fused, and/or otherwise bonded together around the periphery in order to create a bag which can hold fluids.

That the first component part and the second component part can be engaged with the tube radially can be understood to mean that a motion is used to engage the first component part or the second component part with the tube which motion has at least a radial motion component with respect to a longitudinal axis of the tube.

In other words, the motion for engaging the first component part or the second component part can have additionally have axial and/or azimuthal motion component.

That the first component part and/or the second component part can be engaged with the tube can be understood to mean that the first component part or the second component part can be at least partially brought into contact with the tube. However, in accordance with the invention this is not absolutely necessary. For example, it would be possible that the crimping sleeve has slightly larger inner diameter than the outer diameter of the tube. In such embodiments contact between the crimping sleeve and the tube may only be established once the crimping action is exerted onto the crimping sleeve and subsequently onto the tube.

That the first component part and/or the second component part can be engaged with the tube from different sides can be understood to mean that the first component part and the second component part can be distanced from each other so that the tube can - broadly speaking - be arranged at least partly between the first component part and the second component part, and the first component part and the second component part can then be engaged with the tube by a motion towards each other. In other words, the sides do not have to be diametrically opposite each other.

That the first component part and the second component part are separate from each other can be understood to mean that the first component part can be moved relative to the second component part before being crimped. In preferred embodiments the first component part and the second component part can be moved freely relative to each other before being crimped.

In other embodiments according to the invention there can for example be a hinge between the first component part and the second component part so that the first component part and the second component part can be engaged with the tube by a closing motion of the hinge.

In certain embodiments the crimping sleeve can have more than the first component part and the second component part - i.e., a third component part, a fourth component part and so on. For these further component parts the aforesaid regarding engagement, radial engagement, different sides, and separate component parts can be true.

Protection is also sought for an arrangement comprising a tube and a crimping sleeve according to the invention.

Protection is furthermore sought for a method for filling a container, in particular a single use bag, with a fluid, in particular a biopharmaceutical fluid, wherein at least the following steps are carried out: connecting a tube to the container and a bulk source for the fluid, at least partially filling the container with the fluid, and subsequently performing the method according to the invention.

Further advantageous embodiments are defined in the dependent claims.

The first component part and the second component part together can form the complete crimping sleeve.

As mentioned before, embodiments of the invention are however also conceivable where there are more than two component parts which together make up the crimping sleeve according to the invention.

The crimping sleeve can have a base shape of a cylindrical shell.

The first component part and/or the second component part can have a base shape of cylindrical half shell.

If there are more than two component parts making up the crimping sleeve the base shape can be an aliquot ratio of the complete circular shell, e.g., a third of the full shell if there are three component parts.

The base areas of such cylindrical shells and/or half shells preferably correspond to the cross-sectional area of the tube.

The base areas of such cylindrical shells and/or half shells and/or the cross-sectional area of the tube can be circular.

The first component part and the second component part can contact each other when engaged with the tube, preferably on contacting surfaces arranged such that the tube is between the contacting surfaces.

The first component part and/or the second component part can comprise connecting elements for connecting the first component part and the second component part to each other.

The connecting elements can comprise
- a first hook with a free end on the first component part
- a second hook on the second component part adapted to engage with a hooked portion of the first hook or of the first component part
- a third hook on the second component part adapted to engage with the free end of the first hook

The first hook can also be arranged on the second component part, and the second and third hook can be arranged on the first component part.

The first hook, second hook, and/or third hook can be elongated in a longitudinal direction of the crimping sleeve.

These connecting elements can be configured for a snap on connection and/or for a - preferably positive lock - connection which can be created by movement of the first component part relative to the second component part, preferably longitudinally along the tube.

The movement of the first component part relative to the second component part longitudinally along the tube can preferably be a sliding movement.

The first component part and/or the second component part and/or the crimping sleeve can comprise and/or be made from brass, preferably with a Nickel coating.

The Nickel coating can be a chemical Nickel coating and/or a galvanic Nickel coating.

The arrangement according to the invention can further comprise:
- a crimping tool for directly or indirectly exerting a crimping action on the crimping sleeve and/or
- an intermediary sleeve to be disposed between the crimping sleeve and the crimping tool.

The intermediary sleeve could also be called crimping tool insert.

The intermediary sleeve can be made from steel (preferably 1.2379) .

The intermediary sleeve can preferably have a Rockwell hardness greater than 55 HRC, preferably 59 HRC or more.

The crimping tool can for example make use of the general principle of pliers or tongs, i.e., there can be two elongated handles which cause the crimping action on the crimping sleeve or the intermediary sleeve when squeezed together.

The crimping tool and/or the intermediary sleeve can comprise a crimping protrusion which faces the tube and which is disposed transversally, preferably at a right angle, with respect to a longitudinal axis of the tube, the crimping sleeve, the intermediary sleeve, and/or the crimping tool.

The crimping tool and/or the intermediary sleeve can also comprise a crimping recess generally corresponding in shape with the crimping protrusion.

As mentioned before, the intermediary sleeve can comprise a first intermediary component and a separate second intermediary component.

In preferred embodiments the crimping sleeve can have an inner diameter between 0,5 cm (preferably 0,6 cm) to 4 cm (preferably 3,8 cm) .

Further details and advantages of the invention are apparent from the figures and the accompanying description of the figures. The figures show:
- Fig. 1: an embodiment of a crimping sleeve according to the invention,
- Fig. 2: an embodiment of a crimping sleeve according to the invention,
- Fig. 3: an example of an engagement motion according to the invention,
- Fig. 4: an example of an engagement motion according to the invention,
- Fig. 5: an embodiment of an arrangement according to the invention,
- Fig. 6: an embodiment of an arrangement according to the invention,
- Fig. 7: an embodiment of a crimping sleeve according to the invention together with an embodiment of an intermediary sleeve,
- Fig. 8: a detailed view of the connecting elements of the embodiment of Fig. 1, and
- Fig. 9a to 9c: schematic diagrams of method steps according to the invention.

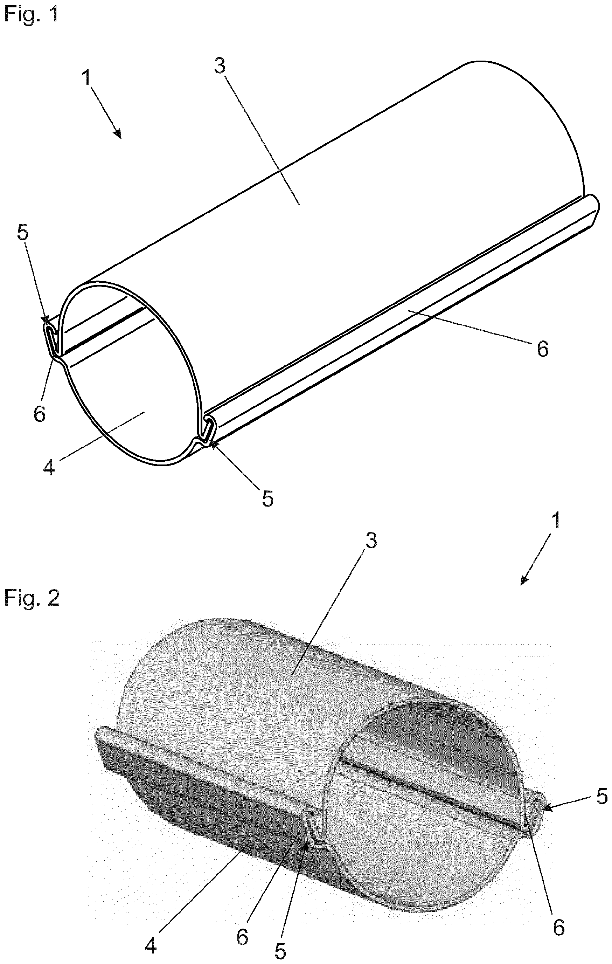

Figures 1 ad 2 show embodiments of a crimping sleeve 1 according to the invention.

It comprises a first component part 3 and a second component part 4, which together form the complete crimping sleeve 1.

The first component part 3 and the second component part 4 are shown in a state in which they are attached to each other using connecting elements 6 in the form of interlocking hooks.

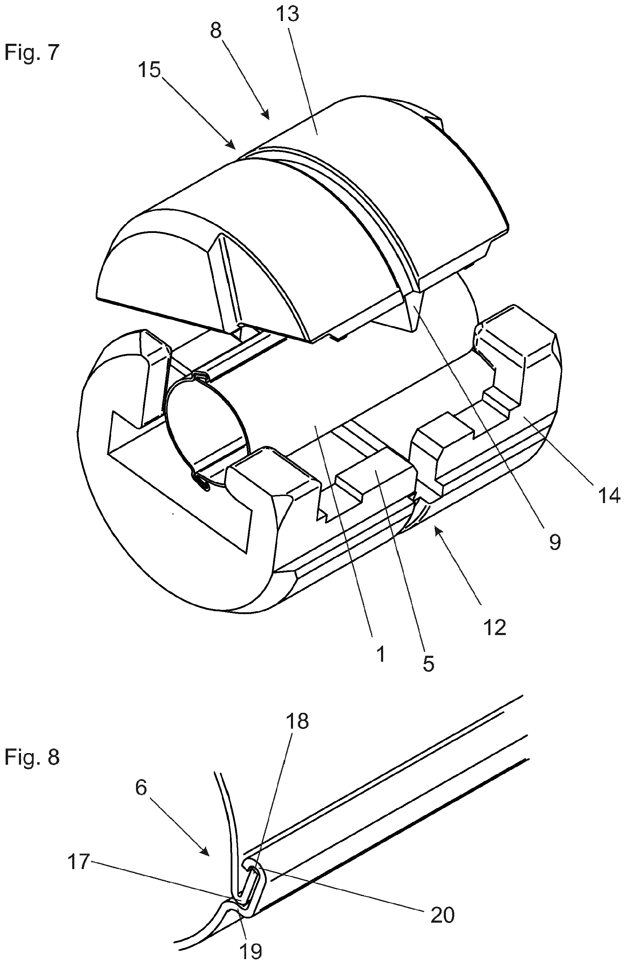

For details regarding the connecting elements 6 it is referred to Fig. 8 and the corresponding description below.

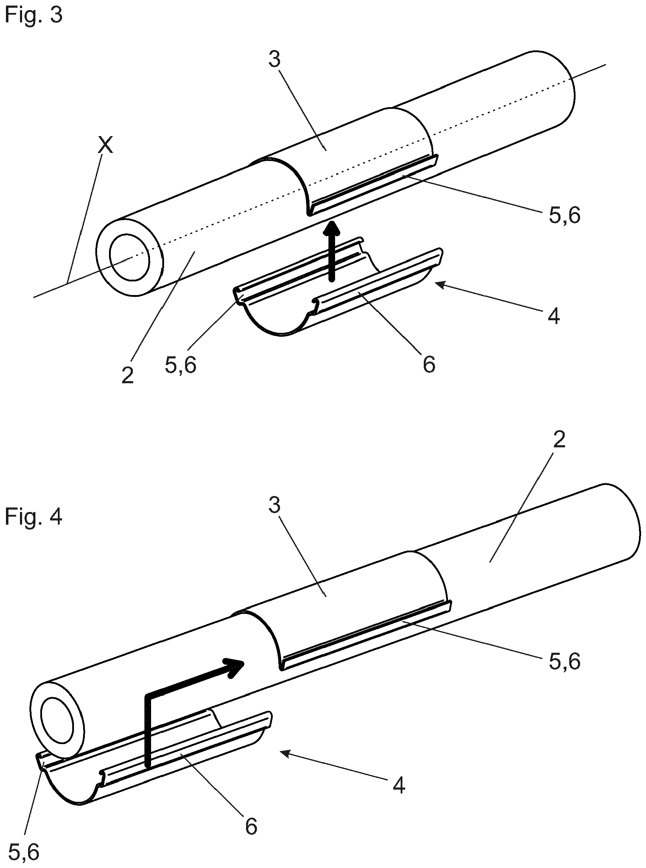

The first component part 3 and the second component part 4 can be disconnected from each other completely and can then be engaged with a tube 2, see also Figures 3, 4, and 5.

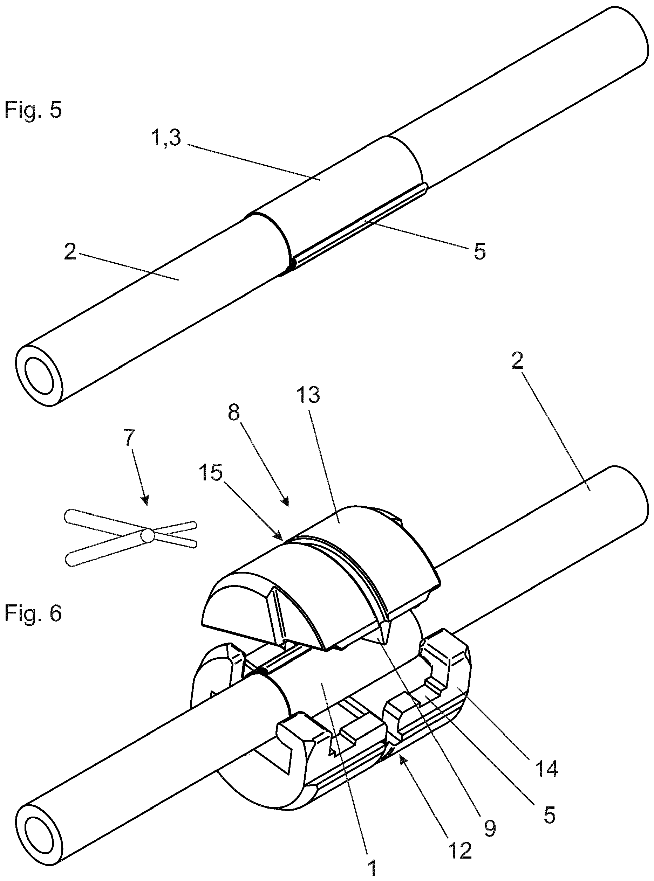

In this embodiment the first component part 3 and the second component part 4 contact each other on contacting surfaces 5 which are on two sides of the tube 2 when the crimping sleeve 1 is engaged with the tube 2, see also Fig. 5.

The first component part 3 and the second component 4 part are made from brass with a Nickel coating.

The crimping sleeve 1 has a base shape of a cylindrical shell.

The first component part 3 and the second component 4 part have a base shape of cylindrical half shell.

The base area in this case is circular in order to correspond to the cross section of the tube 2 for which the crimping sleeve 1 is used, see for example Fig. 5.

Fig. 3 shows an embodiment of an engagement motion with which the embodiments from Fig. 1 and 2 can be engaged with the tube 2.

The first component part 3 is set radially onto the tube 2 from a first side thereby engaging with the tube 2. The first component part 3 is depicted in this state in Fig. 3.

The second component part 4 is set also onto the tube 2 from an opposite side with a radial motion.

This motion is visualised with a thick arrow.

The motion is radial with respect to the longitudinal axis X of the tube 2, the

At the end of the motion the second component part 4 engages with the tube 2.

At the same time the connecting elements 6 in the form of interlocking hooks engage with each other with a snap on action such that contact between the first component part 3 and the second component part 4 is established at contact surfaces 5.

The result is the arrangement depicted in Fig. 5 comprising the tube 2 and the crimping sleeve 1.

Fig. 4 shows an alternative embodiment to Fig. 3. Instead of directly snapping the second component part 4 onto the first component part 3 for engaging with the tube 2 the second component part 4 is engaged with the tube 2 with an axial offset relative to the first component part 3.

Subsequently, the second component part 4 is slid down the tube 2 towards the first component part 3 such that the interlocking hooks of the connecting elements 6 slide into each other.

The combination of the engagement and sliding motion is visualised with a thick arrow.

Again, the result is the arrangement depicted in Fig. 5.

In the embodiments of the Figures 1, 2, and 5 the connecting elements 6 cause a positive lock connection between the first component part 3 and the second component part 4.

In a next step the crimping action can be performed.

For example, it is possible to use crimping tools 7 to directly crimp the crimping sleeve 1. Such crimping tools 7, for instance making use of the general principle of pliers, are in and of themselves known in the prior art.

Another embodiment is depicted in Fig. 6. An intermediary sleeve 8 (itself an embodiment of a crimping sleeve 1 according to the invention) is used together with a crimping tool 7 (shown symbolically).

The intermediary sleeve 8 comprises a first intermediary component 13 and a separate second intermediary component which can be engaged with the crimping sleeve.

Analogously to the first component part 3 and the second component part 4 of the crimping sleeve 1 the first intermediary component 13 and the second intermediary component 14 have contacting surfaces 5.

Statements regarding the first component part 3 and the second component part 4, in particular regarding engagement motions, different sides, connecting elements 6, cross sections, and so on, are analogously true regarding the first intermediary component 13 and the second intermediary component 14.

The intermediary sleeve 8 comprises a crimping protrusion 9 which faces the tube 2 and which is disposed at a right angle with respect to a longitudinal axis X of the tube 2 and the crimping sleeve 1.

The intermediary sleeve 8 also comprises a crimping recess 12 generally corresponding in shape with the crimping protrusion 8.

The contacting surfaces 5 of the intermediary sleeve 8 are stepped in order to automatically position the first intermediary part 13 and the second intermediary part 14 correctly along the longitudinal axis X.

It is noteworthy, that in this embodiment the crimping sleeve 1 and the intermediary sleeve 8 are positioned relative to each other so that the connecting elements 6 of the crimping sleeve 1 are roughly in a central position in relation to the first intermediary part 13 and the second intermediary part 14. In other words. The tube 2 together with the crimping sleeve 1 has been turned by about 90° around the longitudinal axis X before engaging the first intermediary part 13 and the second intermediary part 14 of the intermediary sleeve 8.

The intermediary sleeve 8 can comprise an alignment recess 15. The crimping tool 7 can be configured with an alignment protrusion corresponding to the alignment recess 15. The alignment recess 15 together with the alignment protrusion allow for automatic alignment correction between the crimping tool 7 and the intermediary sleeve 8.

Alternatively or additionally, there could be an alignment recess 15 on the crimping tool 7 and an alignment protrusion on the intermediary sleeve 8.

Fig. 7 depicts an enlarged view of Fig. 6 where the tube 2 and the crimping tool 7 are not shown.

All features described in connection with the crimping sleeve 1 can also be realised in connection with the intermediary sleeve 8, and vice versa.

Fig. 8 shows an enlarged view of the connecting elements 6 in the form of interlocking hooks used on the first component part 3 and the second component part 4 from the embodiment of Fig. 1.

The interlocking hooks or connecting elements 6 comprise
- a first hook 17 with a free end 18 on the first component part 3
- a second hook 19 on the second component part 4 adapted to engage with a hooked portion of the first hook 17
- a third hook 20 on the second component part 4 adapted to engage with the free end 18 of the first hook 17.

The first hook 17, second hook 19, and third hook 20 are elongated in a longitudinal direction (i.e., along the longitudinal axis X) of the crimping sleeve 1.

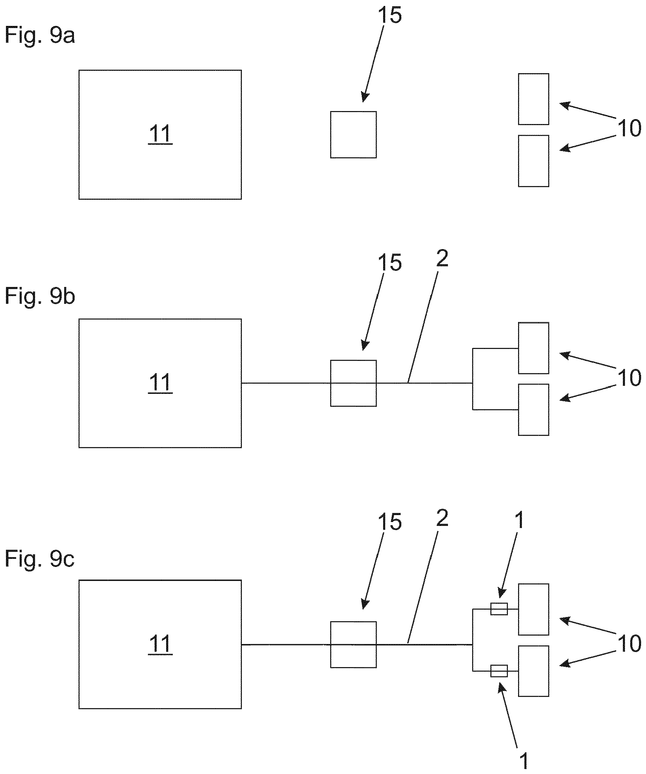

Fig. 9a to 9c show schematic diagrams of method steps according to the invention.

Fig. 9a depicts a state in which there is present a bulk source 11 for biopharmaceutical liquid, a pump 15 and containers 10.

The pump in this case is a peristaltic pump 15.

Instead of the pump 15 it would also be possible to take advantage of a height difference between the bulk source 11 and the containers 10. However, in preferred embodiment a pump 15 is utilised.

The containers 10 in this embodiment are Single Use Bags, although the invention can also be practiced with containers 10 in the form of bottles or the like.

As shown in Fig. 9b a tube 2 is used to connect the bulk source 11 with the containers 10 (here via the peristaltic pump 15).

As can be seen the tube 2 has a single branch on the side of the bulk source 11 and splits into several branches near the containers 10, i.e., the invention is not limited to single-branched tubes 2.

As is clear for persons skilled in the art it is purely exemplary that there are two branches and two containers 10. The invention can be practiced with any number of containers 10 and corresponding branches of the tube 2, such as for example ten or twenty containers 10 and the corresponding number of branches.

The tube 2 can also be called "manifold".

The tube 2 in this case is made from silicone and is flexible to a certain degree.

The flexibility allows the peristaltic pump 15 to pump the biopharmaceutical liquid through the tube 2 into the containers 10 by peristaltic action on the tube 2.

Once the containers 10 are filled to the desired degree (i.e., completely or partially), the containers 10 are to be sealed.

For this, the crimping sleeves 1 according to the invention are engaged with the tube 2 at the locations indicated in Fig. 9c.

The tube 2 can then be crimped and sealed with a crimping tool 7 and optionally an intermediate sleeve 8 in a manner which is in principle known in the prior art.

After sealing the tubes 2 near the containers 10 the tube can be severed on the far side of the containers 10.

The sealed containers 10 with the sealed remnant tube 2 can then be frozen, transported, and/or stored as desired in the further process.

### List of reference numerals:

- 1: crimping sleeve
- 2: tube
- 3: first component part
- 4: second component part
- 5: contacting surfaces
- 6: connecting elements
- 7: crimping tool
- 8: intermediary sleeve
- 9: crimping protrusion
- 10: container
- 11: bulk source
- 12: crimping recess
- 13: first intermediary component
- 14: second intermediary component
- 15: alignment recess
- 16: pump
- 17: first hook
- 18: free end of first hook
- 19: second hook
- 20: third hook

- X: longitudinal axis

## Claims

1. Crimping sleeve for crimping, in particular sealing, a tube (2), in particular for crimping a tube (2) for conveying a biopharmaceutical fluid, **characterised in that** the crimping sleeve (1) comprises a first component part (3,13) and a separate second component part (4,14), wherein the first component part (3,13) and the second component part (4,14) can be engaged with the tube (2) radially from different sides.

2. Crimping sleeve according to claim 1, **characterised in that** the first component part (3,13) and the second component part (4,14) together form the complete crimping sleeve (1,8).

3. Crimping sleeve according to one of the preceding claims, **characterised in that** the crimping sleeve (1,8) has a base shape of a cylindrical shell.

4. Crimping sleeve according to one of the preceding claims, **characterised in that** the first component part (3,13) and/or the second component part (4,14) has/have a base shape of cylindrical half shell.

5. Crimping sleeve according to one of the preceding claims, **characterised in that** the first component part (3,13) and the second component part (4,14) contact each other when engaged with the tube (2), preferably on contacting surfaces (5) arranged such that the tube (2) is between the contacting surfaces (5).

6. Crimping sleeve according to one of the preceding claims, **characterised in that** the first component part (3,13) and/or the second component part (4,14) comprise connecting elements (6) for connecting the first component part (3,13) and the second component part (4,14) to each other.

7. Crimping sleeve according to claim 6, **characterised in that** the connecting elements (6) are configured for a snap on connection and/or for a - preferably positive lock - connection which can be created by movement of the first component part (3,13) relative to the second component part (4,14) longitudinally along the tube (2).

8. Crimping sleeve according to claim 6 or claim 7, **characterised in that** the connecting elements (6) comprise:
- a first hook (17) with a free end (18) on the first component part (3,13)
- a second hook (19) on the second component part (4,14) adapted to engage with a hooked portion of the first hook (17) or of the first component part (3,13)
- a third hook (20) on the second component part (4,14) adapted to engage with the free end (18) of the first hook (17)

9. Crimping sleeve according to one of the previous claims, **characterised in that** the first component part (3,13) and/or the second component part (4,14) and/or the crimping sleeve (1,8) is made from brass, preferably with a Nickel coating.

10. Arrangement comprising a tube (2) and a crimping sleeve (1,8) according to one of the previous claims.

11. Arrangement according to claim 10, **characterised in that** the arrangement further comprises
- a crimping tool (7) for directly or indirectly exerting a crimping action on the crimping sleeve (1,8) and/or
- an intermediary sleeve (8) to be disposed between the crimping sleeve (1) and the crimping tool (7).

12. Arrangement according to claim 11, **characterised in that** the crimping tool (1) and/or the intermediary sleeve (8) comprise a crimping protrusion (9) which faces the tube (2) and which is disposed transversally with respect to a longitudinal axis (X) of the tube (2), the crimping sleeve (1), the intermediary sleeve (8), and/or the crimping tool (7).

13. Arrangement according to claim 11 or 12, **characterised in that** the intermediary sleeve (8) comprises a first intermediary component (13) and a separate second intermediary component (14) .

14. Method for crimping, in particular sealing, a tube (2), in particular for crimping a tube (2) for conveying a biopharmaceutical fluid, in particular using a crimping sleeve (1) according to one of the claims 1 to 9, wherein at least the following steps are carried out:
- radially engaging a first component part (3,13) of a crimping sleeve (1) with the tube (2),
- radially engaging a second component part (4,14) of the crimping sleeve (1,8) with the tube (2) from a different side than the first component part (3,13), and
- exerting a crimping action onto the crimp sleeve (1,8), thereby crimping - and preferably sealing - the tube (2).

15. Method for filling a container (10), in particular a single use bag, with a fluid, in particular a biopharmaceutical fluid, wherein at least the following steps are carried out: connecting a tube (2) to the container (10) and a bulk source (11) for the fluid, at least partially filling the container (10) with the fluid, and subsequently performing the method of claim 14.
